# EUROPEAN PATENT APPLICATION

(11) **EP 3 834 835 A1**
(43) Date of publication of application: **16.06.2021**
(21) Application number: 19848283.8
(22) Date of filing: 09.08.2019
(51) Int. Cl.: A61K 35/76, A61P 17/00, A61P 31/04

(54) **BACTERIOPHAGE PREPARATION**

(30) Priority: 10.08.2018 JP 2018151950
(71) Applicant: Meiji Seika Pharma Co., Ltd., Tokyo 104-8002 (JP); Incorporated Educational Institution Rakuno Gakuen, Ebetsu-shi, Hokkaido, 069-8501 (JP)
(72) Inventor: IWANO Hidetomo, Ebetsu-shi, Hokkaido 069-8501 (JP); HIGUCHI Hidetoshi, Ebetsu-shi, Hokkaido 069-8501 (JP); TAMURA Yutaka, Ebetsu-shi, Hokkaido 069-8501 (JP); USUI Masaru, Ebetsu-shi, Hokkaido 069-8501 (JP); FUJIKI, Jumpei, Ebetsu-shi, Hokkaido 069-8501 (JP)
(74) Representative: J A Kemp LLP
(86) International application number: PCT/JP2019/031559
(87) International publication number: WO 2020/032232

(57) **Abstract**

It has been found that a bacteriophage specified by accession number NITE BP-693 and a bacteriophage specified by accession number NITE BP-694 have lytic activity against Staphylococcus hyicus, Staphylococcus chromogenes, and the like, which has made it possible to provide a composition for lysing these staphylococci, a composition for treating or preventing a disease caused by these staphylococci, and the like.

## Description

### [Technical Field]

The present invention relates to a composition for lysing staphylococci (such as Staphylococcus hyicus and Staphylococcus chromogenes), containing a bacteriophage as an active ingredient having a property of lysing the staphylococci. Further, the present invention relates to a prophylactic or therapeutic use of the composition for diseases caused by the staphylococci. The present invention also relates to a composition for detecting the staphylococci, containing the bacteriophage.

### [Background Art]

Types of staphylococci such as Staphylococcus hyicus (S. hyicus) and Staphylococcus chromogenes (S. chromogenes) cause various diseases in humans and animals . Exudative epidermitis is known as one of the skin diseases caused by S. hyicus, S. chromogenes, and the like (NPLs1 to 3 and PTL 1). Exudative epidermitis is called "sooty disease, " in which piglets are in a state of being covered with soot all over the body, and often develop in a maternal abdomen, and in a severe state, death occurs at 20 to 30%. The treatment thereof involves use of antibacterial agents, disinfectants, steroidal agents, and the like showing effectiveness on them (NPL 4). However, in recent years, there are many problems such as the increase of drug-resistant bacteria resulting in the inability to exert the effect of antibacterial agents, and the damage of the skin by disinfectants resulting in the worsening of symptoms, and there is no effective treatment method.

Bacteriophage is a general term for viruses that infect bacteria, and when infecting a host bacterium, it proliferates within the bacterium. The proliferated daughter phages are released from the cell wall of the bacterium to the outside of the bacterium, and the bacterium is killed by lysis. In addition, bacteriophages have advantages that they do not affect the microbiota other than the host pathogen, that they are easy to proliferate, that a large amount of bacteriophage can be prepared at low cost, and the like.

In view of the above, efforts have been made to use bacteriophage as a bactericidal agent, and some research results have been reported on the sterilization of Staphylococcus aureus (S. aureus) using bacteriophage. For example, as bacteriophages having the property of lysing S. aureus, the present inventors have found and reported in PTL 2 bacteriophages specified by accession number NITE BP-693 and accession number NITE BP-694.

However, there are no reports of bacteriophages having lytic activity against S. hyicus or S. chromogenes . In addition, bacteriophages have a problem that the host specificity is so high that bacteria of the same species are not infected if the strains are different. Therefore, an effective bacteriophage is required for preventing or treating diseases caused by these staphylococci.

### [Citation List]

### [Patent Literature]

[PTL 1] US Patent No. 8084040
[PTL 2] Japanese Patent No. 5720045

### [Non Patent Literature]

[NPL 1] H. C. Wegener, et al., Can. J. Ver. Res., 1993, 57, 119-125
[NPL 2] L. O. Andresen, et al., Vet. Microbiol., 2005, 105, 291-300
[NPL 3] Aiden P. Foster Vet. Dermatol . , 2012, 23, 342-351
[NPL 4] C. L'Ecuyer and D. C. Alexander, Can. Ver. J. 1969, 10(9), 227-234

### [Summary of Invention]

### [Technical Problem]

An object of the present invention is to find a bacteriophage having a property of lysing S. hyicus, S. chromogenes, and the like, and to provide a composition for lysing the staphylococci, or a composition for preventing or treating a disease caused by the staphylococci, containing the bacteriophage as an active ingredient.

### [Solution to Problem]

The present inventors have previously found bacteriophages specified by accession number NITE BP-693 and accession number NITE BP-694 as bacteriophages having a property of lysing S. aureus (PTL 2).

This time, regarding these bacteriophages, the lytic activity against a plurality of S. hyicus strains and a plurality of S. chromogenes strains isolated from pigs suffering from exudative epidermitis was evaluated. As a result, it has been surprisingly found that both bacteriophages have high lytic activity against a plurality of S. hyicus strains and a plurality of S. chromogenes strains, despite the fact that bacteriophages usually have extremely high host specificity.

Furthermore, it has also been found that both bacteriophages have high lytic activity against other staphylococci (Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus). Thus, the present invention has been completed. Specifically, the present invention has the following configuration.
(1) A composition for lysing at least one staphylococcus bacterium selected from the group consisting of Staphylococcus hyicus, Staphylococcus chromogenes, Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus, comprising at least one bacteriophage selected from the group consisting of a bacteriophage specified by accession number NITE BP-693 and a bacteriophage specified by accession number NITE BP-694 as an active ingredient.
(2) A composition for preventing or treating a disease caused by at least one staphylococcus bacterium selected from the group consisting of Staphylococcus hyicus, Staphylococcus chromogenes, Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus, comprising at least one bacteriophage selected from the group consisting of a bacteriophage specified by accession number NITE BP-693 and a bacteriophage specified by accession number NITE BP-694 as an active ingredient.
(3) The composition according to (2), which is an injectable composition, an oral composition, or an external composition.
(4) The composition according to (2), which is an external composition and is administered to a subject by sprinkling, dipping, spreading, or dropping.
(5) A composition for detecting at least one staphylococcus bacterium selected from the group consisting of Staphylococcus hyicus, Staphylococcus chromogenes, Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus, comprising at least one bacteriophage selected from the group consisting of a bacteriophage specified by accession number NITE BP-693 and a bacteriophage specified by accession number NITE BP-694, or a part thereof.

### [Advantageous Effects of Invention]

The present invention makes it possible to provide a composition for lysing S. hyicus, S. chromogenes, and the like, and a composition for preventing or treating diseases caused by these staphylococci.

In particular, in the present invention, the bacteriophage as the active ingredient of these compositions exhibits infectivity and high lytic activity against a plurality of strains of these staphylococci. Therefore, it is possible to efficiently sterilize these staphylococci.

Furthermore, the present invention also makes it possible to provide a composition for detecting these staphylococci by using the host-specific adsorptivity of the above bacteriophage.

In addition, if a corresponding host exists, a bacteriophage infects the host and proliferates explosively. However, the bacteriophage cannot proliferate on its own in the absence of a host. Therefore, if the particular host is completely lysed and killed by the bacteriophage, the bacteriophage cannot proliferate either. In addition, it does not infect and affect other bacteria either. Therefore, for example, once the composition of the present invention is sprinkled on a pig breeding facility or the like to eradicate the staphylococci, it is not necessary to then perform any treatment to remove the bacteriophage. From the above respects, the present invention has an advantage that it is safe and easy to use without adversely affecting the environment and other bacteria.

### [Description of Embodiments]

### (Composition for Lysis)

As shown in Examples described later, it has been clarified that the bacteriophages having lytic activity against Staphylococcus aureus (the bacteriophage specified by accession number NITE BP-693 and the bacteriophage specified by accession number NITE BP-694) also exhibit lytic activity against Staphylococcus hyicus, Staphylococcus chromogenes, and the like.

Therefore, the present invention provides a composition for lysing at least one staphylococcus bacterium selected from the group consisting of Staphylococcus hyicus, Staphylococcus chromogenes, and the like, comprising at least one bacteriophage selected from the group consisting of a bacteriophage specified by accession number NITE BP-693 and a bacteriophage specified by accession number NITE BP-694 as an active ingredient (hereinafter also referred to as the "composition for lysis").

The subjects of the composition of the present invention, namely Staphylococcus hyicus (S. hyicus), Staphylococcus chromogenes (S. chromogenes), Staphylococcus pseudintermedius (S. pseudintermedius), Staphylococcus epidermidis (S. epidermidis), Staphylococcus haemolyticus (S. haemolyticus), Staphylococcus saprophyticus (S. saprophyticus), Staphylococcus sciuri (S. sciuri), Staphylococcus warneri (S. warneri), Staphylococcus auricularis (S. auricularis), Staphylococcus capitis (S. capitis), Staphylococcus cohnii (S. cohnii), Staphylococcus condimenti (S. condimenti), Staphylococcus felis (S. felis), Staphylococcus hominis (S. hominis), Staphylococcus intermedius (S. intermedius), Staphylococcus schleiferi (S. schleiferi), Staphylococcus simulans (S. simulans), Staphylococcus xylosus (S. xylosus), and Staphylococcus argenteus (S. argenteus), are species of the eubacteria which are Gram-positive cocci belonging to the genus Staphylococcus (hereinafter, these staphylococci are also collectively referred to as the "staphylococci according to the present invention").

In addition, "lysing" means that in the infection of a bacteriophage, the cell membrane and cell wall of the bacterium are destroyed, the cytoplasm is released (eluted) to the outside of the cell, and the bacterium is killed.

One of the bacteriophages as an active ingredient of the composition of the present invention is a bacteriophage deposited on December 25, 2008 under accession number NITE BP-693 at NITE Patent Microorganisms Depositary, Department of Biotechnology, National Institute of Technology and Evaluation (postal code 292-0818, 2-chome-5-8 Kazusakamatari, Kisarazu, Chiba, Japan). Note that the present inventors have named this bacteriophage "ϕSA012."

The other of the bacteriophages according to the present invention is a bacteriophage deposited on December 25, 2008 under accession number NITE BP-694 at NITE Patent Microorganisms Depositary, Department of Biotechnology, National Institute of Technology and Evaluation (postal code 292-0818, 2-chome-5-8 Kazusakamatari, Kisarazu, Chiba, Japan). The present inventors have named this bacteriophage "ϕSA039."

Observation using a transmission electron microscope revealed that both ϕSA012 and ϕSA039 are about the same size as phage T4 and slightly longer. Further, since ϕSA012 and ϕSA039 have a contractile sheath, it is presumed that they belong to the Myoviridae family. For other structures and properties of the bacteriophages according to the present invention, refer to the description in PTL 2.

The bacteriophages according to the present invention can be obtained by making a purchase request to the depository institution to which they are deposited. In addition, they can also be obtained, for example, by a conventional method such as a plaque assay·separation method described in PTL 2, which separates and purifies running sewage of a general household, wastewater collected from a sewage treatment facility, or the like or biological samples such as blood, nasal cavity, pharynx, skin, and intestinal tract derived from animals infected with S. aureus. In addition, the bacteriophages according to the present invention can be allowed to proliferate by a general bacteriophage proliferation method such as the plate lysate method described in PTL 2.

The composition of the present invention may contain the above-mentioned bacteriophage. Examples thereof include a bacteriophage solution obtained by the above-mentioned proliferation method, a bacteriophage solution separated from a culture residue by centrifugation or the like.

Details of the bacteriophage contained in the composition of the present invention are as described above. In addition, the bacteriophage contained in the composition of the present invention may be one kind (strain) or a bacteriophage mixture (phage cocktail) of ϕSA012 and ϕSA039. Further, it may also contain an additional bacteriophage. The additional bacteriophage is not particularly limited, and examples thereof include bacteriophages against bacteria different from staphylococci according to the present invention (for example, S. aureus), and may be not only lytic but also lysogenic bacteriophage.

The concentration of bacteriophage in the composition for lysis of the present invention is not particularly limited and can be appropriately adjusted by those skilled in the art depending on the mode of use thereof. For example, when used in a liquid dosage form (such as a liquid antibacterial agent) described later, it is preferably 1 × 10³ to 1 × 10¹³ PFU/ml (PFU: plaque forming unit), more preferably 1 × 10⁵ to 1 × 10¹¹ PFU/ml, and further preferably 1 × 10⁸ to 1 × 10¹⁰ PFU/ml.

The composition for lysis of the present invention can lyse the staphylococci according to the present invention by the above-mentioned bacteriophage, and thus can take the form of a pharmaceutical composition. Examples of pharmaceutical composition include pharmaceutical compositions for preventing or treating diseases caused by the staphylococci (pharmaceutical products such as therapeutic drugs, preventive drugs, and symptom improving drugs), and pharmaceutical compositions for killing or exterminating the staphylococci (such as bactericidal agents, for example, pharmaceutical products such as disinfectants, and quasi-drugs such as medicated soap) .

The composition for lysis of the present invention is not limited to pharmaceutical compositions, but can also be used in the form of food for the prevention or improvement of diseases caused by the staphylococci. Examples of such food and drink compositions include food with health claims such as food for specified health uses, food with nutrient function claims, and food with functional claims as well as animal feeds, nutritional supplements, dietary supplements, nutritionally prepared food, food for therapy, or food additives.

In addition, the composition for lysis of the present invention may take the form of a composition for reducing the number of the staphylococci (such as a bacteria removing agent) or a composition for suppressing the proliferation of the staphylococci (such as an antibacterial agent). Furthermore, the composition for lysis of the present invention may also take the form of a composition (such as a reagent) for lysing the staphylococci for research purposes (for example, in vitro or in vivo experiments).

The form of the composition for lysis of the present invention can be appropriately changed depending on the subject (such as an administration subject), and for example, solid, semi-solid, and liquid are employed.

For example, when the composition for lysis of the present invention is used as a pharmaceutical composition, a bacteria removing agents, an antibacterial agent, or the like, it is prepared in a form suitable for the subject (preparation form, dosage form). Examples of the dosage form include oral compositions (internal compositions), external compositions, and injectable compositions, and more specific examples of the oral compositions include solid preparations such as tablets, chewable tablets, effervescent tablets, troches, drops, capsules (hard capsules, soft capsules), boluses, granules, pulvis, pills, fine granules, powders, dry syrups, soaking agents decoctions, and liposome preparations, semi-solid preparations such as licking agents, jellies, and chewing gum agents, and liquid preparations such as syrups, drinks, suspensions, oils, emulsions, and alcohols. In addition, examples of external compositions include solid preparations such as suppositories, poultices, and plasters, semi-solid preparations such as ointments, creams, mousses, sheet preparations, resin preparations, gel preparations (such as nasal gel preparations), and inhaler preparations, and liquid preparations such as nasal drops, eye drops, liniments, lotions, sprays, aerosols, spot-on preparations, pore-on preparations, transdermal patches, nenlization agents, suspensions, oils, and emulsions. Further, the liquid preparations may take a form where it is contained in a woven fabric, a knitted fabric, a non-woven fabric, or the like by a method such as dipping. In addition, the bacteria removing agents, the antibacterial agent, and the like may also take the form of a wettable powder, a flowable agent, a heat transpiration agent, a fuming agent, a smoking agent, a ULV agent, a pellet agent, and the like.

These dosage forms can be appropriately prepared by those skilled in the art using pharmacologically acceptable auxiliary components (such as medium and carrier (such as solid carrier, liquid carrier, and gaseous carrier)). Examples of the auxiliary components include aqueous media such as buffer solutions (such as phosphate buffer solutions), physiological saline, and distilled water, non-aqueous media such as polyethylene glycol, propylene glycol, and ethyl oleate, and commonly used ones such as surfactants, vegetable oils, solvents, bases, emulsifiers, suspending agents, matrix-forming agents, excipients, vehicles, thickeners, pressure-sensitive adhesive agents, spreading agents, binders, diluents, lubricants, protective agents, viscosity accelerators, wicking agents, stabilizers, buffers, isotonic agents, chelating agents, disintegrants, pH adjusters, solubilizers, wetting agents, colorants, dyes, pigments, flavoring agents, aromatic agents, corrigents, sweeteners, flavor modifiers, antiseptics, viscous agents, preservatives, and antioxidants. Note that these auxiliary components must be compatible with the bacteriophage as an active ingredient. In addition, the composition of the present invention may be appropriately mixed with endolysin (an enzyme possessed by a bacteriophage for digesting the cell wall of a host during lysis) in order to further improve lytic properties.

The composition for lysis of the present invention may further contain an additional drug, or may be used in combination. When used in combination, they may be administered at the same time or before or after. In addition, a drug can be added to the bacteriophage of the present invention.

The "additional drug" is not particularly limited as long as the bacteriophage according to the present invention does not lose its infectivity and lytic activity on the staphylococci according to the present invention, but according to the mode of use, it is possible to appropriately select and use drugs commonly used for various purposes such as antibacterial agents, anti-inflammatory agents, immunosuppressive agents, infectious disease preventive agents, infectious disease therapeutic agents, insecticides, anthelmintics, antifungal agents, and antiviral agents.

Examples of "antibacterial agents" include, but are not limited to, β-lactam-based, aminoglycoside-based, lincomycin-based, fosfomycin-based, tetracycline-based, chloramphenicol-based, macrolide-based, ketolide-based, polypeptide-based, glycopeptide-based, streptogramin-based, quinolone-based, and oxazolidinones.

Examples of the "anti-inflammatory agents" include steroidal agents and non-steroidal agents. Examples of the "steroidal agents" include corticosteroids, and more specific examples include, but are not limited to, prednisolone, beclomethasone, betamethasone, fluticasone, dexamethasone, hydrocortisone, prednicarbate, mometasone, and derivatives thereof. Examples of the "non-steroidal agents" include, but are not limited to, salicylic acid-based, propionic acid-based, acetic acid-based, oxime-based, pilin-based, non-pilin-based compounds, and COX-2 inhibitors.

Examples of the "immunosuppressive agents" include, but are not limited to, antimetabolites, alkylating agents, microtubule synthesis inhibitors, lymphocyte signal transduction inhibitors, cytokine inhibitors, and antibodies.

### (Lysis Method)

In the present invention, the lysis of the staphylococci according to the present invention (such as sterilization, eradication, and suppression of proliferation) is not particularly limited, and can be carried out by bringing the bacteriophage according to the present invention or the composition for lysis of the present invention into contact with the subject for lysis treatment.

Specifically, the present invention also provides a method for lysing at least one of the staphylococci according to the present invention in a subject by bringing the bacteriophage according to the present invention or the composition for lysis of the present invention into contact with the subject.

The "subject" is not particularly limited, and includes animals exemplified in the following (such as treatment method), substances in which the staphylococci can exist, and environments (for example, food, equipment and devices used for processing, cooking, and storing food, food processing factories, environments to which food is exposed, breeding facilities for animals (such as pigs), and the like).

The method for "contact" is not particularly limited, and the bacteriophage according to the present invention can be brought into contact with the subject by, for example, sprinkling, dipping, spreading, dropping, or the like described later. In addition, the time of contact with the subject is not particularly limited, and may be a sufficient time for the bacteriophage according to the present invention to lyse the staphylococci according to the present invention in the subject and prevent the proliferation of the staphylococci.

### (Treatment Method and the Like)

As described above, the bacteriophage according to the present invention exhibits infectivity and high lytic activity against a plurality of strains of the staphylococci according to the present invention, and can kill these staphylococci. Therefore, by using the bacteriophage according to the present invention, it is possible to prevent, treat, or ameliorate diseases caused by these staphylococci.

Therefore, the present invention also provides a method for preventing or treating a disease of a subject caused by at least one of the staphylococci according to the present invention by administering the bacteriophage according to the present invention or the pharmaceutical composition of the present invention to the subject.

The "subject" for treatment or the like is not particularly limited as long as it can be infected with the staphylococci according to the present invention, and may be a human or a non-human animal. Examples of the non-human animals include livestock, pets, and laboratory animals, and more specific examples include mammals such as pigs, bovines, horses, sheep, goats, monkeys, rabbits, cats, dogs, minks, mice, rats, hamsters, and guinea pigs, and birds such as poultry.

The "disease caused by staphylococci" is not particularly limited as long as it is caused by at least one of the staphylococci according to the present invention, and examples thereof include skin infections (such as dermatitis, exudative epidermitis, folliculitis, footpad dermatitis, footpad dermatitis, poultry staphylococci disease, pyoderma, pustular dermatitis, purulent dermatitis, skin abscess, and skin infection), urinary tract infections (such as cystitis, lower urinary tract infection, and urethral infection), bacteremia (such as catheter-related bacteremia, bacteremia, artificial joint infection, sepsis, and septic polyarthritis), bone and joint infections (such as osteomyelitis, arthritis, osteitis and arthritis, and bone infection and joint infection), genital infections (such as vaginitis, metritis, and pyometra (uterus cystoma)), breast infections (such as mammitis and mastitis), soft tissue infections (such as soft tissue infection), ear and nose infections (such as otitis, otitis externa, otitis media, and sinusitis), wound infections (such as wound infection and surgical wound infection), respiratory organ infections (such as respiratory tract infection and pneumonia), eye infections (such as endophthalmitis, conjunctivitis, eye infections including eyelids and conjunctiva, eye infection, and endophthalmitis after eye surgery), central nervous system infections (such as meningoencephalitis and meningoencephalitis), and food poisoning.

In addition, examples of diseases caused by the staphylococci in various animals are as follows.

**[Table 1]**

| Infection Subject | Disease | |
|---|---|---|
| | Classification | Disease Example |
| Pig | Skin Infection (Including Dermatitis) | Dermatitis, Skin Infection, Exudative Epidermitis |
| | Urinary Tract Infection | Urinary Tract Infection, Cystitis |
| | Bacteremia | Bacteremia |
| | Bone and Joint Infections | Bone and Joint Infections |
| | Genital Infection | vaginitis, Metritis |
| Bovine | Breast Infection | Mammitis, Mastitis |
| | Skin Infection (Including Dermatitis) | Skin Infection, Dermatitis |
| | Urinary Tract Infection | Urinary Tract Infection |
| Poultry | Skin Infection (Including Dermatitis) | Skin Infection, Dermatitis, Exudative Epidermitis, Folliculitis, Footpad Dermatitis, Skin Purulent Disease, Footpad Dermatitis, Staphylococcusis, Impetigo |
| | Bone and Joint Infections | Bone and Joint Infections, Osteomyelitis |

**[Table 2]**

| Infection Subject | Disease | |
|---|---|---|
| | Classification | Disease Example |
| Dog | Skin Infection (Including Dermatitis) | Pyoderma, Pyoderma Tumor, Pustular Dermatitis, Skin Infection, Dermatitis, Purulent Dermatitis |
| | Soft Tissue Infection | Soft Tissue Infection |
| | Ear and Nose Infections | Otitis Externa, Otitis Media |
| | Wound Infection | Wound Infection |
| | Bone and Joint Infections | Bone and Joint Infections |
| | Respiratory Organ Infection | Respiratory Tract Infection |
| | Eye Infection | Eye Infections Including Eyelids and Conjunctiva |
| | Genital Infection | Genital Infection, Pyometra (Uterus Cystoma) |
| | Central Nervous System Infection | Meningoencephalitis |

**[Table 3]**

| Infection Subject | Disease | |
|---|---|---|
| | Classification | Disease Example |
| Cat | Skin Infection (Including Dermatitis) | Skin Infection, Dermatitis, Pyoderma Tumor, Pustular Dermatitis |
| | Otitis | Otitis Media, Otitis Externa |
| | Urinary Tract Infection | Cystitis (Lower Urinary Tract Infection) |
| | Genital Infection | Pyometra (Uterus Cystoma) |
| | Bone and Joint Infections | Bone and Joint Infections |
| | Respiratory Organ Infection | Respiratory Tract Infection |
| | Wound Infection | Wound Infection |
| | Eye Infection | Eye Infections Including Eyelids and Conjunctiva, Conjunctivitis |

**[Table 4]**

| Infection Subject | Disease | |
|---|---|---|
| | Classification | Disease Example |
| Goat | Skin Infection (Including Dermatitis) | Skin Infection, Dermatitis |
| | Breast Infection | Mammitis |
| Horse | Skin Infection (Including Dermatitis) | Skin Infection, Purulent Dermatitis, Dermatitis |
| Sheep | Breast Infection | Mammitis |
| Mink | Skin Infection (Including Dermatitis) | Purulent Dermatitis, Dermatitis |

**[Table 5]**

| Infection Subject | Disease | |
|---|---|---|
| | Classification | Disease Example |
| Human | Skin Infection (Including Dermatitis) | Dermatitis, Skin Infection, Skin Abscess, Skin Purulent Disease |
| | Wound Infection | Surgical Wound Infection, Wound Infection |
| | Bacteremia | Catheter-Related Bacteremia, Bacteremia, Artificial Joint Infection, Sepsis |
| | Bone and Joint Infections | Bone and Joint Infections, Osteomyelitis |
| | Ear and Nose Infections | Otitis, Sinusitis |
| | Central Nervous System Infection | Meningitis |
| | Eye Infection | Eye Infection, Endophthalmitis After Eye Surgery, Endophthalmitis |
| | Urinary Tract Infection | Urinary Tract Infection |
| | Soft Tissue Infection | Soft Tissue Infection |
| | Food Poisoning | Food Poisoning |

In addition, examples of diseases caused by various staphylococci are as follow.

**[Table 6]**

| Staphylococcus Bacteria | Disease | Literature |
|---|---|---|
| Staphylococcus chromogenes | Pig: 7, 18 Exudative Epidermitis | 7: Anderson. (Vet. Microbiol., 105, 291-300 (2005)) |
| | Bovine: 18 Mammitis | 18: Bryan Markey, 105-119. (2013) In Clinical Vet. Microbiology, 2nd ed., Bryan Markey, Finola Leonard, Marine Archambault, Ann Cullinane, Dores Maguire |
| | Horse and Cat: 18 Dermatitis | |
| Staphylococcus hyicus | Pig: 2, 17, 18 Exudative Epidermitis; 8 Arthritis; 8 Cystitis; 17, 18 Septic Polyarthritis; 18 Meningitis; 18 Vaginitis; 8 Miscarriage | 2: Japanese Unexamined Patent Application Publication No. Hei 7-118158 |
| | Bovine: 8 Dermatitis; 8, 17 Mammitis; 18 Mastadenitis; 18 Skin Infection; 17 Scabies | 8: Hideaki Shimada (SDI, 43 (2010) → with reference document) |
| | Goat: 8 Dermatitis, 8 Mammitis | 17: Akira Shimizu (Journal of Veterinary Medical Science 42(2) 77-89 (1989)) |
| | Poultry: 17 Exudative Epidermitis; 8 Folliculitis; 8 Dermatitis; 8 Buttock Crust; 8 Footpad Dermatitis; 19 Osteomyelitis | 18: Bryan Markey, 105-119. (2013) In Clinical Vet. Microbiology, 2nd ed., Bryan Markey, Finola Leonard, Marine Archambault, Ann Cullinane, Dores Maguire |
| | Human: 8 Skin Abscess; 8 Rash | 19: Yutaka Ueda (Journal of Veterinary Medical Science 60, 519-522 (2007)) |
| | Horse: 17 Dermatitis; 18 Skin Infection | |

**[Table 7]**

| Staphylococcus Bacteria | Disease | Literature |
|---|---|---|
| Staphylococcus pseudintermedius | Dog: 1 Pyoderma; 18 Pyoderma Tumor, Pustular Dermatitis, Uterus Cystoma, Otitis Externa, Infections Including Respiratory Tract, Bone, Joint, Wound, Eyelid, and Conjunctiva | 1: Kanako Ishihara (MP AGRO Journal, 29-33 (2013)) |
| | Cat: 18 Pyoderma Tumor, Pustular Dermatitis, Uterus Cystoma, Infections Including otitis Externa, Respiratory Tract, Bone, Joint, Wound, Eyelid, and Conjunctiva | 18: Bryan Markey, 105-119. (2013) In Clinical Vet. Microbiology, 2nd ed., Bryan Markey, Finola Leonard, Marine Archambault, Ann Cullinane, Dores Maguire |
| | Human: 23 Bacteremia, Brain Abscess and Lung Cancer, Endocarditis, Surgical Wound Infection, Sinusitis, Catheter-Related Bacteremia | 23: Staphylococcus Bacteria Infection 5 |
| | | - Surgical Wound Infection, Sinusitis, Catheter-Related Bacteremia: |
| | | Journal of Antimicrobial Chemotherapy, Volume 66, Issue 12, December 2011, Pages 2705-2714. |
| Staphylococcus epidermidis | Poultry: 17 Skin Purulent Disease | 17: Akira Shimizu (Journal of veterinary Medical Science 42(2) 77-89 (1989)) |
| | Bovine: 17 Mammitis | 9: Ken Kikuchi (Journal of the Japanese Society of Internal Medicine, 97, 11, 2673-2677 (2008)) |
| | Human: 17 Skin Purulent Disease; 9 Bacteremia; 9, 11, 17 Endocarditis; 9 Vascular Graft-Stent Infection; 9 Post-Cardiovascular Surgery Infection; 9 VP-Shunt Related Meningitis; 9 CAPD Peritonitis; 9 Artificial Joint Infection (Bacteremia); 9 Osteomyelitis; 9 Endophthalmitis After Eye Surgery; 11, 17 Urinary Tract Infection; 11 Catheter Infection (Bacteremia); 11 Sepsis | 11: Masahiro Furumoto (Shinshu Medical Journal 53(4), 209-220 (2005)) |
| | | Human Bacteremia, 9, 11 |
| | | Human Artificial Joint Infection, Osteomyelitis, Endophthalmitis After Eye Surgery: 9 |
| | | Human Endocarditis: 9, 13 |
| | | Vascular Graft-Stent Infection, Post-Cardiovascular Surgery Infection, VP-Shunt Related Meningitis, CAPD Peritonitis, Artificial Joint Infection, Osteomyelitis, Endophthalmitis After Eye Surgery: 9 |

**[Table 8]**

| Staphylococcus Bacteria | Disease | Literature |
|---|---|---|
| Staphylococcus haemolyticus | Human: Sepsis, Peritonitis; 10 Otitis; 10 Urinary Tract Infection, Endocarditis; 16 Catheter Infection | 10: Takeuchi F., (J. Bacteriol., 187, 7292-7308 (2005)) |
| | | 16: Yoshida Pharmaceutical (Y's Letter 17 (2003)) |
| Staphylococcus saprophyticus | Human: 4, 11, 17 Urinary Tract Infection, Sexually Transmitted Disease, Prostatitis; 17 Wound Infection, Sepsis, Catheter Infection, Endocarditis | 4: Toshiro Okuda (Journal of the Japanese Association for Infectious Diseases, 56, 2, 111-117 (1982)) |
| | | 11: Masahiro Furumoto (Shinshu Medical Journal 53(4), 209-220 (2005)) |
| | | 17: Akira Shimizu (Journal of Veterinary Medical Science 42(2) 77-89 (1989)) |

**[Table 9]**

| Staphylococcus Bacteria | Disease | Literature |
|---|---|---|
| Staphylococcus sciuri | Pig: 3 Exudative Epidermitis | Exudative Epidermitis |
| | Bovine: 14 Mammitis | 3: Chen, S. (PLoS ONE, 1-6 (2007)) and Larissa A. Z. Condas, J Dairy Sci. 2017 Jul; 100(7): 5613-5627 |
| | Human: 14 Endocarditis; 14 Urinary Tract Infection; 14 Wound Infection; 14 Endophthalmitis, Pelvic Inflammatory Disease, Peritonitis, Septic Shock | |
| | | Urinary Tract Infection |
| | | 14: Rivera, M (Perit Dial Int. 34(4) 469-470 (2014) and PLoS One, 2007; 2(1): e147, Published online 2007 Jan 10, doi: 10.1371/journal.pone.0000147 |
| | | Wound Infection |
| | | 14: Rivera, M (Perit Dial Int. 34(4) 469-470 (2014) and PLoS One, 2007; 2(1): e147, Published online 2007 Jan 10, doi: 10.1371/journal.pone.0000147 |
| | | Bovine Mammitis |
| | | Larissa A. Z. Condas, J Dairy Sci. 2017 Jul; 100(7): 5613-5627 |

**[Table 10]**

| Staphylococcus Bacteria | Disease | Literature |
|---|---|---|
| staphylococcus warneri | Human: 4, 24 Urinary Tract Infection, Endocarditis and Sepsis; 16 Catheter Infection, Vertebral Osteomyelitis, Ventriculoperitoneal Shunt Infection; 24 Meningitis, Spontaneous Miscarriage, Orthopedic Infection, ventricular Shunt Infection, Endocarditis | 4: Toshiro Okuda (Journal of the Japanese Association for Infectious Diseases, 56, 2, 111-117 (1982)) |
| | Bovine: Spontaneous Miscarriage, 24 Urinary Tract Infection; 24 Meningitis, Orthopedic Infection, Ventricular Shunt Infection, Endocarditis | 16: Yoshida Pharmaceutical (Y's Letter 17 (2003)) |
| | Dog: Meningoencephalitis | 24 WIKIPEDIA |
| | | Human Meningitis: 24 and Rev Inst Med Trop Sao Paulo, Jun; 52(3): 169-70 |
| Staphylococcus auricularis | Human: Opportunistic Infection and Sepsis | |
| Staphylococcus capitis | Human: endocarditis, Sepsis, Artificial Joint Infection | |
| Staphylococcus cohnii | Poultry: 20 Staphylococcus Bacteria Disease (Spinal Damage) | 20: Rikako Ariyoshi (Avian Disease Information Vol. 11 (issued on October 14, 2014)) |
| | Human: 16 Catheter Infection, Meningitis | 16: Yoshida Pharmaceutical (Y's Letter 17 (2003)) |

**[Table 11]**

| Staphylococcus Bacteria | Disease | Literature |
|---|---|---|
| Staphylococcus condimenti | Human: 12 Catheter Infection (Bacteremia), Bacteremia, Soft Tissue Infection | 12: Misawa, Y., (New Microbes New Infect. 3, 18-20 (2015) |
| | | Bacteremia |
| | | New Microbes New Infect. 2015 Jan; 3: 18-20 |
| | | Published online 2014 Oct 17, doi: 10.1016/j.nmni.2014.10.002 |
| Staphylococcus felis | Cat: 18 Otitis Media, Otitis Externa, Abscess; 18 Dermatitis, Skin Infection; 18 Cystitis (Lower Urinary Tract Infection); 18 Conjunctivitis | 18: Markey, B. Clinical Vet. Microbiology, 105-119 (2013) |
| | | Otitis Externa: Can Vet J Volume 32, May 312-313 (1991) |
| | | Skin Infection, Cystitis: Wikipedia https://en.wikipedia.org/wiki/Staphylococcus felis |

**[Table 12]**

| Staphylococcus Bacteria | Disease | Literature |
|---|---|---|
| Staphylococcus hominis | Human: 11 Urinary Tract Infection; 16 Catheter Infection, Sepsis, Eye Infection, Endocarditis, Peritonitis, Joint Infection | 11: Masahiro Furumoto (Shinshu Medical Journal 53(4), 209-220 (2005)) |
| | | 16: Yoshida Pharmaceutical (Y's Letter 17 (2003)) |
| | | Eye Infection: |
| | | http://www.jarmam.gr.jp/situmon/staph-aure.html |
| Staphylococcus intermedius | Dog: 1, 17 Pyoderma; 17 Middle and External Otitis; 17 Genital Infection; 17 Wound Infection, Skin Infection, Soft Tissue Infection | 1: Kanako Ishihara (MP AGRO Journal, 29-33 (2013)) |
| | Cat: 17 Middle and External Otitis | 17: Akira Shimizu (Journal of Veterinary Medical Science 42(2) 77-89 (1989)) |
| | Horse and Mink: 17 Purulent Dermatitis | |
| | Human: Soft Tissue Infection, Brain Abscess | Dog Skin Infection and Soft Tissue Infection Nancy Wang, Infectious Disease Reports 2013; 5: e3 6-11 |
| Staphylococcus schleiferi | Dog: Pyoderma | 16: Yoshida Pharmaceutical (Y's Letter 17 (2003)) |
| | Human: 16 Catheter Infection | |
| | | Pyoderma |
| | | Thein Swe, J Investig Med High Impact Case Rep. 2016 Jul-Sep; 4(3): 1-3 2324709616671148 |

**[Table 13]**

| Staphylococcus Bacteria | Disease | Literature |
|---|---|---|
| Staphylococcus simulans | Bovine: Mammitis | Bovine Mammitis |
| | Sheep: Mammitis | JAAD Case Rep. 2016 Nov; 2(6): 428-429 |
| | Poultry: 13 Footpad Dermatitis; 20 Staphylococcus Bacteria Disease | |
| | Human: Endocarditis and Sepsis, Pleural Abscess, Epidermal Nodule, Ulcer Lesion, Psoriasis, Osteomyelitis | Sheep Mammitis |
| | | G. C. FTHENAKIS, Epidemiol. Infect. (1994), 112, 171-176 |
| | | 13: Shin'ichiro Hashimoto (Journal of the Japan veterinary Medical Association, 65, 199-203 (2012)) |
| | | 20: Rikako Ariyoshi (Avian Disease Information Vol. 11 (issued on October 14, 2014)) |
| | | Human Epidermal Nodule, Ulcer Lesion: JAAD Case Rep. 2016 Nov; 2(6): 428-429 |

**[Table 14]**

| Staphylococcus Bacteria | Disease | Literature |
|---|---|---|
| Staphylococcus xylosus | Mouse: Dermatitis | 4: Toshiro Okuda (Journal of the Japanese Association for Infectious Diseases, 56, 2, 111-117 (1982)) |
| | Human: Erythema Nodosum; 4 Urinary Tract Infection; 16 Catheter Infection, Acute Pyelonephritis | 16: Yoshida Pharmaceutical (Y's Letter 17 (2003)) |
| Staphylococcus argenteus | Human: Sepsis, Purulent Lymphadenitis, Food Poisoning | |

The "Literature" shown in Tables 6 to 14 discloses the relationship between various staphylococci and the diseases caused by infecting animals.

The "administration" method of the bacteriophage or the like according to the present invention is not particularly limited, and examples of the administration include transdermal administration, intradermal administration, subcutaneous administration, intravascular injection (intravenous administration, intra-arterial administration), local administration (such as local injection), oral administration, transmucosal (for example, nasal) administration, intraperitoneal administration, tracheobronchial administration, rectal administration and intramuscular administration, and administration by infusion. Such administration can be carried out by appropriately employing the above-mentioned dosage form. The dose is appropriately adjusted according to the type, age (week age), body weight, symptom, health condition, and the like of the subject (animal).

For example, when the pharmaceutical composition of the present invention is used as an external composition, the bacteriophage according to the present invention at a concentration of about 1 × 10⁶ to 1 × 10¹² PFU/ml can be contained in the external composition and administered, although it depends on the type of the subject and the like as described above.

Examples of the method for administering the external composition include sprinkling, dipping, spreading, and dropping. More specific examples of the dipping include bathing the subject in the external composition or water containing the same. Examples of the sprinkling include spraying, nebulization, misting, showering, and dusting the external composition on the subject. Examples of the spreading include coating and wiping the external composition on the subject, or allowing the external composition to be contained in a molded product such as a strip, a plate, a band, a collar, an earmark, a limb band, or a labeling device, and bringing the subjects (animals) equipped with the molded product into contact with each other, to thereby attach and disperse the external composition to the animals. Examples of the dropping include poring and spotting the external composition on the subject.

More specifically, for example, the method for coating or nebulizing the external composition on the subject includes, but is not limited to, coating or nebulizing the bacteriophage according to the present invention at a concentration of about 1 × 10⁴ to 1 × 10¹² PFU, preferably about 1 × 10⁵ to 1 × 10¹² PFU per 5 cm² of the affected area, although it depends on the type and condition of the subject as described above.

When the pharmaceutical composition of the present invention is used as an injectable composition, for example, the bacteriophage according to the present invention at a concentration of about 1 × 10⁶ to 1 × 10¹² PFU/ml can be contained in the injectable composition and administered, although it depends on the type of the subject and the like as described above.

In addition, for example, the method for directly administering the injectable composition to the affected area includes, but is not limited to, injecting into the affected area the bacteriophage according to the present invention at a concentration of about 1 × 10⁴ to 1 × 10¹² PFU, preferably about 1 × 10⁵ to 1 × 10¹² PFU, although it depends on the type and condition of the subject as described above.

Furthermore, for example, the method for administering the injectable composition into the blood includes, but is not limited to, intravascular injection at a concentration of about 1 × 10⁴ to 1 × 10¹² PFU/Kg, although it depends on the type and condition of the subject as described above.

Note that since bacteriophage is a virus that has high host specificity and infects only the host bacteria, it has almost no toxicity when administered to animals. Therefore, it is possible to administer a large amount of bacteriophage depending on the type of the subject animal, the condition of the affected area, and the like. For example, if the animal for administration is a large animal, a larger amount of bacteriophage can be administered.

### <Composition for Detecting Staphylococci>

Bacteriophage is known to lyse the host by specifically recognizing and adsorbing to the host. Then, the host can be detected by using the formation of a complex between the bacteriophage and the host due to this specific adsorptivity as an index (see Japanese Unexamined Patent Application Publication No. 2004-283003).

Therefore, the present invention also provides a composition for detecting at least one staphylococcus bacterium selected from the staphylococci according to the present invention, comprising at least one bacteriophage selected from the group consisting of a bacteriophage specified by accession number NITE BP-693 and a bacteriophage specified by accession number NITE BP-694, or a part thereof.

The bacteriophage contained in the detection composition of the present invention is as described above, but may be a part of the bacteriophage as long as it has adsorption specificity to at least one of the staphylococci according to the present invention. In addition, the bacteriophage or a part thereof may be bound with a labeling substance. By detecting the labeling substance, it becomes possible to directly detect the bacteriophage bound to staphylococci.

The "labeling substance" is not particularly limited as long as it can bind to bacteriophage and can be detected by a chemical or optical method, and examples thereof include fluorescent proteins such as green fluorescent protein (GFP), allophycocyanin (APC), and phycoerythrin (R-PE), enzymes such as alkaline phosphatase (ALP), horseradish peroxidase (HRP), and β-galactosidase (β-gal), radioisotopes such as ¹²⁵I, fluorescent dyes such as fluorescein isothiocyanate (FITC) and rhodamine isothiocyanate (RITC), color labeling substances such as colloidal metals and colored latex, avidin, and biotin. Note that in the case of using an enzyme as a labeling substance, various detections can be performed depending on the substrate by adding a coloring substrate, a fluorescent substrate, a chemiluminescent substrate, or the like as the substrate. In addition, the binding of the labeling substance may be directly bound to the bacteriophage, or may be indirectly bound via a different substance. Examples of the "different substance" include so-called secondary antibodies. Here, the "secondary antibodies" are antibodies that exhibit specific binding to the bacteriophage according to the present invention, and use of the antibody bound with a labeling substance allows the labeling substance to indirectly bind to the bacteriophage.

The detection composition of the present invention may contain an additional component permitted as the composition in addition to the bacteriophage according to the present invention. Examples of the additional component include carriers, excipients, disintegrants, buffers, emulsifiers, suspending agents, stabilizers, preservatives, antiseptics, physiological saline, labeled compounds, and secondary antibodies.

Further, in addition to the above-mentioned detection composition of the present invention, a substrate necessary for detection of a labeling substance, a positive control or a negative control, a buffer solution used for diluting or washing a sample, or the like can be combined, and it is also possible to form a kit for detecting at least one of the staphylococci according to the present invention. In addition, when an unlabeled bacteriophage is used as a standard, a labeled substance (for example, an antibody) that binds to the bacteriophage can be combined. In addition, the detection kit can include instructions for use with the kit.

Moreover, the detection composition of the present invention can detect at least one of the staphylococci according to the present invention, and thus can also be used as a composition for diagnosing a disease caused by the staphylococci or as a diagnostic kit containing the composition.

### <Method for Detecting Staphylococci>

As described above, the host can be detected by using the formation of a complex between the bacteriophage and the host as an index.

Therefore, the present invention may also provide a method for detecting a Staphylococcus bacterium, comprising the steps of bringing a sample into contact with a bacteriophage according to the present invention or a part thereof, and detecting a complex of the bacteriophage or the part thereof with at least one Staphylococcus bacterium selected from the group consisting of staphylococci according to the present invention.

The "sample" is not particularly limited as long as the staphylococci can be present, and examples thereof include the tissues or cells derived from the above-mentioned animals or washing solutions thereof, food, equipment and devices used for processing, cooking, and storing food, food processing factories, environments to which food is exposed, breeding facilities for animals (such as pigs), and the like, or washing solutions thereof.

Known immunological methods can be appropriately used for "contact" between the bacteriophage or a part thereof and the sample and for "detection" of the complex. Examples of immunological methods include IMS, immunofluorescence, ELISA, and immunochromatography. Note that those skilled in the art can understand that in these immunological methods, the bacteriophage according to the present invention is used instead of the antibody to detect the staphylococci.

In addition, the detection method of the present invention can detect at least one of the staphylococci according to the present invention, and thus can also be used as a method for diagnosing a disease caused by the staphylococci.

### [Examples]

Hereinafter, the present invention is described in more detail based on Examples, but the present invention is not limited to the following Examples.

The present inventors have previously succeeded in isolating the bacteriophages (ϕSA012 and ϕSA039) specified by accession number NITE BP-693 and accession number NITE BP-694 as bacteriophages having the property of lysing S. aureus.

In the tests shown below, the present Examples evaluated these bacteriophages in terms of lytic activity against S. hyicus and S. chromogenes isolated from pigs suffering from exudative epidermitis.

### (Example 1)

### (Lytic Activity Test of Bacteriophages ϕSA012 and ϕSA039 against S. hyicus and S. chromogenes Derived from Exudative Epidermitis)

The strains to be examined for lytic activity used were a plurality of S. hyicus strains and S. chromogenes strains separated and selected by a conventional method using mannitol agar medium from pigs with exudative epidermitis bred in multiple different places, which were stored at the Pharmaceutical Research Center of Meiji Seika Pharma Co., Ltd.

The S. hyicus strains and the S. chromogenes strains to be examined for lytic activity were extracted, suspended in LB medium, and then cultured in an incubator (37°C) for 9 hours to enrich bacteria. Note that the composition of the LB medium is as follows. Specifically, in the case of LB liquid medium, it contains 0.5 w/v% of NaCl, 0.5 w/w% of yeast extract, and 1 w/v% of Polypeptone. In the case of LB agar medium, 1.5% agar is further contained in addition to the above composition.

The bacteriophages ϕSA012 and ϕSA039 were added to SM buffer solution (0.1mMNaCl, 8 mM MgSO₄ ·7H₂O, 50 mM Tris-HCl, 0.1% gelatin) and stirred to obtain a suspension of the bacteriophages.

LB top agar (concentration of agar: 0.5%) was kept at 45°C using a heat block, and the strains after enrichment were added. The LB top agar and the strains were thoroughly stirred, and then uniformly poured onto the LB agar medium and allowed to stand at room temperature for about 30 minutes for complete solidification.

Onto the solidified LB top agar (soft agar medium), 5 µl of ϕSA012, 5 µl of ϕSA039, or 5 µl of SM buffer (control) was added (spotted) dropwise, followed by culture overnight (37°C for 10 hours) in an incubator. Then, plaques generated at the spots on the soft agar medium were detected.

The lytic activity of the bacteriophage was determined by the transparency of the formed plaque. That is, if the bacteriophage at the spot has lytic activity against the S. hyicus strains or S. chromogenes strains used, S. hyicus or S. chromogenes cannot proliferate after overnight culture but clear spots should form on the soft agar medium. If the plaque is partially turbid or very turbid, that indicates the lytic activity of the host (S. hyicus or S. chromogenes) due to the bacteriophage is low.

Table 15 presents the lytic activity of the bacteriophages evaluated in this manner against various staphylococci strains. In Tables 15 to 20, the case where the plaque generated at each spot is completely clear is indicated by "C," the case of turbid is indicated by "T, " and the case of no plaque (No Spot) is indicated by "N. " In the tables, "Specimen Number" indicates the number assigned to each strain in the present Example. "Strain Source" indicates the source of each strain used in the present Example. The sources "MSP Seibutsuken," "Rakuno Gakuen University," and "JCM" are, respectively, the Pharmaceutical Research Center of Meiji Seika Pharma Co., Ltd., Professor Usui at the Laboratory of Food Microbiology and Food Safety of Rakuno Gakuen University, and Japan Collection of Microorganism (Microbe Division of RIKEN BioResource Center). Moreover, the "Registration Number" indicates a registration number (such as a catalog number) at the source of each strain used in the present Example.

**[Table 15]**

| Staphylococcus Bacteria | Specimen Number | Strain Source | Registrati on Number | Lytic Activity Evaluation by Plaque | |
|---|---|---|---|---|---|
| | | | | phiSA012 | phiSA039 |
| Staphylococcus chromogenes | c-11 | MSP Seibutsuken | 15541 | C | C |
| | c-12 | MSP Seibutsuken | 15541 | C | C |
| | c-13 | MSP Seibutsuken | 15541 | C | C |
| | c-41 | MSP Seibutsuken | 15837 | T | C |
| | c-52 | MSP Seibutsuken | 15750 | C | C |
| Staphylococcus hyicus | h-1 | MSP Seibutsuken | 13970 | C | C |
| | h-3 | MSP Seibutsuken | 12792 | C | T |
| | h-5 | MSP Seibutsuken | 12747 | T | C |
| | h-6 | MSP Seibutsuken | 12261 | C | C |

As is apparent from the results presented in Table 15, both ϕSA012 and ϕSA039 exhibited lytic activity against a plurality of strains of S. hyicus and S. chromogenes.

### (Example 2)

Furthermore, regarding the bacteriophages specified by accession number NITE BP-693 and accession number NITE BP-694 (ϕSA012 and ϕSA039), the same test as described was also carried out to evaluate the lytic activity of Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus. Tables 16 to 20 present the obtained results.

**[Table 16]**

| Staphylococcus Bacteria | Specimen Number | Strain Source | Registrati on Number | Lytic Activity Evaluation by Plaque | |
|---|---|---|---|---|---|
| | | | | phiSA012 | phiSA039 |
| Staphylococcus pseudintermedius | p-1 | Rakuno Gakuen University | MRS37 | C | T |
| | p-2 | Rakuno Gakuen University | MRS57 | T | T |
| | p-3 | Rakuno Gakuen University | MRS64 | T | T |
| | p-4 | Rakuno Gakuen University | MRS84 | C | C |
| | p-6 | Rakuno Gakuen University | SP102 | T | T |
| | p-7 | Rakuno Gakuen University | SP109 | T | T |
| | p-8 | Rakuno Gakuen University | SP119 | T | T |
| | p-9 | Rakuno Gakuen University | SP143 | C | C |
| | p-10 | Rakuno Gakuen University | SP145 | T | T |

**[Table 17]**

| Staphylococcus Bacteria | specimen Number | Strain Source | Registrati on Number | Lytic Activity Evaluation by Plaque | |
|---|---|---|---|---|---|
| | | | | phiSA012 | phiSA039 |
| Staphylococcus epidermidis | e-2 | Rakuno Gakuen University | HP-197I | C | C |
| | e-5 | Rakuno Gakuen University | HB-157IIB | T | T |
| | e-7 | Rakuno Gakuen University | HP-197a | T | T |
| | e-8 | Rakuno Gakuen University | HP-196IIW1 | C | C |
| | e-9 | Rakuno Gakuen University | HP-196IIW2 | T | T |
| | e-10 | Rakuno Gakuen University | HB-147IIB | T | T |
| | e-11 | JCM | 2414 | C | C |
| | e-12 | JCM | 20345 | T | T |

**[Table 18]**

| Staphylococcus Bacteria | Specimen Number | Strain Source | Registrati on Number | Lytic Activity Evaluation by Plaque | |
|---|---|---|---|---|---|
| | | | | phiSA012 | phiSA039 |
| Staphylococcus haemolyticus | ha-1 | Rakuno Gakuen University | HP-104I | T | T |
| | ha-2 | Rakuno Gakuen University | HP-112b | T | T |
| | ha-3 | Rakuno Gakuen University | HP-114a | C | C |
| | ha-6 | Rakuno Gakuen University | HP-207b | C | T |
| | ha-8 | Rakuno Gakuen University | HB-103a | C | T |
| | ha-10 | Rakuno Gakuen University | 37-VT-3a | C | T |
| Staphylococcus saprophyticus | s-1 | Rakuno Gakuen University. | HP-23-1 | T | T |
| | s-2 | Rakuno Gakuen University | HB-40IB | T | T |
| | s-3 | Rakuno Gakuen University | HB-65IB | T | T |
| | s-4 | Rakuno Gakuen University | HB-51a | T | T |
| | s-5 | Rakuno Gakuen University | HB-105b | T | T |
| | s-8 | Rakuno Gakuen University | HB-135b | T | T |

**[Table 19]**

| Staphylococcus Bacteria | Specimen Number | Strain Source | Registrati on Number | Lytic Activity Evaluation by Plaque | |
|---|---|---|---|---|---|
| | | | | phiSA012 | phiSA039 |
| Staphylococcus sciuri | sc-1 | Rakuno Gakuen University | HP-109IIW | T | T |
| | sc-6 | Rakuno Gakuen University | HP-108I | T | T |
| | sc-7 | Rakuno Gakuen University | HB-37IB | T | T |
| | sc-8 | Rakuno Gakuen University | HB-116IIB | T | C |
| | sc-9 | Rakuno Gakuen University | HP-114I | T | T |
| | sc-10 | Rakuno Gakuen University | HP-105IIB | T | T |
| | sc-11 | JCM | 2425 | T | T |
| Staphylococcus warneri | w-3 | Rakuno Gakuen University | HP-106I | T | T |
| | w-4 | Rakuno Gakuen University | HP-112IIB | C | T |
| | w-6 | Rakuno Gakuen University | HP-139b | C | T |
| | w-7 | Rakuno Gakuen University | HB-371B | T | T |
| | w-8 | Rakuno Gakuen University | HP-159b | C | C |
| | w-9 | Rakuno Gakuen University | HP-173a | T | T |
| | w-10 | Rakuno Gakuen University | HP-158a | T | T |
| | w-11 | JCM | 2415 | T | T |

**[Table 20]**

| Staphylococcus Bacteria | Specimen Number | Strain Source | Registration Number | Lytic Activity Evaluation by Plaque | |
|---|---|---|---|---|---|
| | | | | phiSA012 | phiSA039 |
| Staphylococcus auricularis | aur-1 | JCM | 2421 | C | C |
| Staphylococcus capitis | ca-1 | JCM | 2420 | C | N |
| Staphylococcus cohnii | co-1 | JCM | 2417 | C | C |
| Staphylococcus condimenti | con-1 | JCM | 6074 | C | T |
| Staphylococcus felis | f-1 | JCM | 7469 | C | N |
| Staphylococcus hominis | ho-1 | JCM | 31912 | T | N |
| Staphylococcus intermedius | i-1 | JCM | 2422 | T | T |
| Staphylococcus schleiferi | sc-1 | JCM | 7470 | C | T |
| Staphylococcus simulans | si-1 | JCM | 2424 | T | T |
| Staphylococcus xylosus | x-1 | JCM | 2418 | T | T |
| Staphylococcus argenteus | ar-1 | JCM | 31982 | T | T |

As is apparent from the results presented in Tables 16 to 20, ϕSA012 and ϕSA039 generally exhibited lytic activity against the above-mentioned staphylococci, and also generally exhibited lytic activity against a plurality of strains of each Staphylococcus bacterium.

### [Industrial Applicability]

As described above, the present invention makes it possible to lyse Staphylococcus hyicus, Staphylococcus chromogenes, Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus. In particular, the bacteriophage according to the present invention has high lytic activity against a plurality of strains of these staphylococci, and thus can efficiently sterilize the staphylococci. Furthermore, use the host-specific adsorptivity of the bacteriophage makes it possible to detect the staphylococci.

Therefore, the present invention is useful in eradication of the staphylococci, prevention or treatment of diseases caused by the staphylococci, examination of the staphylococci, and the like.

### [Accession Number]

(1) Identification Reference : 003 + ϕSA012
(2) Accession Number: NITE BP-693
(3) Accession Date: December 25, 2008
(4) Depository Institution: National Institute of Technology and Evaluation
(1) Identification Reference : 0031 + ϕSA039
(2) Accession Number: NITE BP-694
(3) Accession Date: December 25, 2008
(4) Depository Institution: National Institute of Technology and Evaluation

## Claims

1. A composition for lysing at least one staphylococcus bacterium selected from the group consisting of Staphylococcus hyicus, Staphylococcus chromogenes, Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus, comprising at least one bacteriophage selected from the group consisting of a bacteriophage specified by accession number NITE BP-693 and a bacteriophage specified by accession number NITE BP-694 as an active ingredient.

2. A composition for preventing or treating a disease caused by at least one staphylococcus bacterium selected from the group consisting of Staphylococcus hyicus, Staphylococcus chromogenes, Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus, comprising at least one bacteriophage selected from the group consisting of a bacteriophage specified by accession number NITE BP-693 and a bacteriophage specified by accession number NITE BP-694 as an active ingredient.

3. The composition according to claim 2, which is an injectable composition, an oral composition, or an external composition.

4. The composition according to claim 2, which is an external composition and is administered to a subject by sprinkling, dipping, spreading, or dropping.

5. A composition for detecting at least one staphylococcus bacterium selected from the group consisting of Staphylococcus hyicus, Staphylococcus chromogenes, Staphylococcus pseudintermedius, Staphylococcus epidermidis, Staphylococcus haemolyticus, Staphylococcus saprophyticus, Staphylococcus sciuri, Staphylococcus warneri, Staphylococcus auricularis, Staphylococcus capitis, Staphylococcus cohnii, Staphylococcus condimenti, Staphylococcus felis, Staphylococcus hominis, Staphylococcus intermedius, Staphylococcus schleiferi, Staphylococcus simulans, Staphylococcus xylosus, and Staphylococcus argenteus, comprising at least one bacteriophage selected from the group consisting of a bacteriophage specified by accession number NITE BP-693 and a bacteriophage specified by accession number NITE BP-694, or a part thereof.
